# EUROPÄISCHE PATENTSCHRIFT

(11) **EP 0 679 650 B1**
(45) Veröffentlichungstag und Bekanntmachung des Hinweises auf die Patenterteilung: **16.10.2002**
(21) Anmeldenummer: 95105542.5
(22) Anmeldetag: 12.04.1995
(51) Int. Cl.: C07D 401/04, A01N 43/56, A01N 43/40

(54) **Substituierte Pyridylpyrazole**
Substituted pyridylpyrazoles
Pyridilpyrazoles substitués

(30) Priorität: 25.04.1994 DE 4414333
(43) Veröffentlichungstag der Anmeldung: 02.11.1995
(73) Patentinhaber: BAYER AG, 51368 Leverkusen (DE)
(72) Erfinder: Stetter, Jörg, Professor Dr., D-42115 Wuppertal (DE); Alig, Bernd, Dr., D-53639 Königswinter (DE); Marhold, Albrecht, Dr., D-51373 Leverkusen (DE); Mencke, Norbert, Dr., D-51381 Leverkusen (DE); Mrusek, Klaus, D-51467 Bergisch Gladbach (DE); Turberg, Andreas, Dr., D-40699 Erkrath (DE)

(56) Entgegenhaltungen:
- EP-A- 0 201 852
- EP-A- 0 207 285
- EP-A- 0 235 628
- EP-A- 0 500 209

## Beschreibung

Die Erfindung betrifft neue substituierte Pyridylpyrazole, mehrere Verfahren zu ihrer Herstellung und ihre Verwendung als Schädlingsbekämpfungsmittel.

Es ist bereits bekannt, dass bestimmte substituierte 1-Arylpyrazole, wie beispielsweise 5-Amino-1-[2,6-dichlor-4-(trifluormethyl)-phenyl]-3-cyano-4-[(trifluormethyl)-sulfinyl]-1H-pyrazol eine gute Wirksamkeit gegen Schädlinge besitzen (vgl. z.B. EP-A 295 117 und EP-A 352 944).

Weiterhin sind zahlreiche substituierte 1-Arylpyrazole beschrieben, die zur Bekämpfung von Schädlingen eingesetzt werden können (vgl. z.B. EP-A 201 852, EP-A 418 016, EP-A 500 209).

Darüber hinaus dienen substituierte 1-Arylpyrazole auch als Zwischenprodukte für die Herstellung von Schädlingsbekämpfungsmitteln (vgl. z.B. EP-A 301 338, EP-A 301 339, EP-A 374 061, EP-A 260 521).

Die Wirkhöhe bzw. Wirkungsdauer der vorbekannten Verbindungen ist jedoch, insbesondere bei bestimmten Insekten oder bei niedrigen Anwendungskonzentrationen, nicht in allen Anwendungsgebieten völlig zufriedenstellend.

Es wurden nun neue substituierte Pyridylpyrazole der allgemeinen Formel (I) 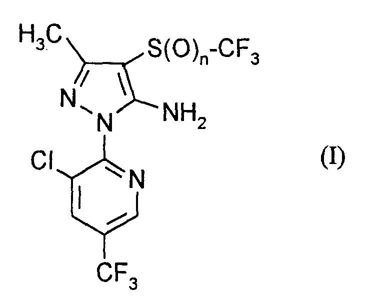 in welcher
- n: für die Zahl 0, 1 oder 2 steht gefunden.

Weiterhin wurde gefunden, daß man die neuen substituierten Pyridylpyrazole der allgemeinen Formel (I) nach einem der im folgenden beschriebenen Verfahren erhält:
a) Man erhält 5-Amino-3-methyl-4-trifluormethylthio-[(3-chlor-5-trifluormethyl)-2-pyridyl]-pyrazol der Formel (Ia) 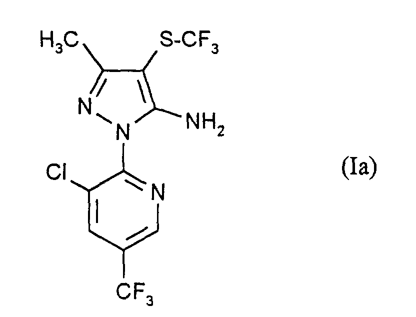 wenn man 5-Amino-3-methyl-[(3-chlor-5-trifluormethyl)-2-pyridyl]-pyrazol der Formel (II) 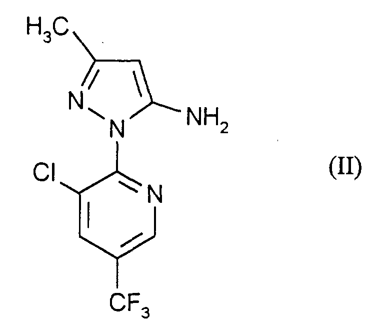 mit Trifluormethylsulfenylhalogeniden der Formel (III)

   CF₃-S-Hal (III)

   in welcher
   - Hal: für Halogen, insbesondere Chlor oder Brom steht,
   gegebenenfalls in Gegenwart eines Verdünnungsmittels und gegebenenfalls in Gegenwart eines Reaktionshilfsmittels umsetzt.
b) Man erhält substituierte Pyridylpyrazole der Formel (Ib) 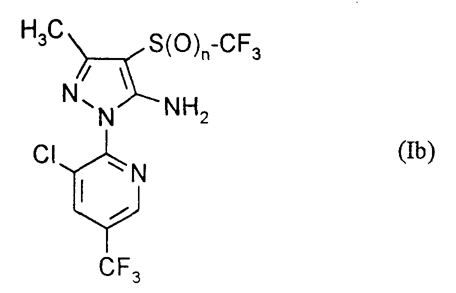 in welcher
   - n: für die Zahl 1 oder 2 steht,
   wenn man 5-Amino-3-methyl-4-trifluormethylthio-[(3-chlor-5-trifluormethyl)-2-pyridyl]-pyrazol der Formel (Ia) 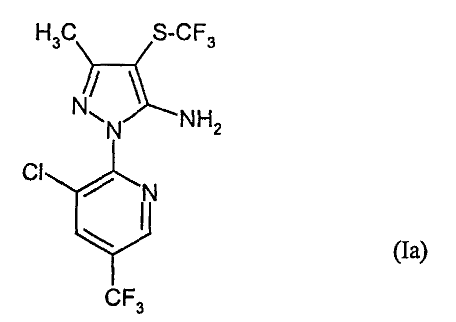 mit Oxidationsmitteln und in Gegenwart eines Verdünnungsmittels und gegebenenfalls in Gegenwart eines Katalysators oxidiert.

Schließlich wurde gefunden, dass die neuen substituierten Pyridylpyrazole der Formel (I) eine sehr gute Wirksamkeit gegen Schädlinge und insbesondere eine sehr gute insektizide und akarizide Wirksamkeit besitzen.

Überraschenderweise zeigen die erfindungsgemäßen Pyridylpyrazole der Formel (I) eine erheblich bessere Wirksamkeit gegenüber tierischen Schädlingen, als die konstitutionell ähnlichen vorbekannten Verbindungen.

Das zur Durchführung des erfindungsgemäßen Verfahrens (a) benötigte 5-Amino-3-methyl-[(3-chlor-5-trifluormethyl)-2-pyridyl]-pyrazol der Formel (II) kann nach allgemein bekannten Verfahren und in analoger Weise erhalten werden, wenn man 3-Aminocrotonitril der Formel (IV) 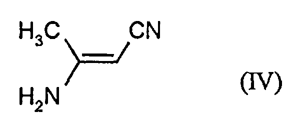 und
3-Chlor-5-(trifluormethyl)-2-pyridyl-hydrazin der Formel (V) 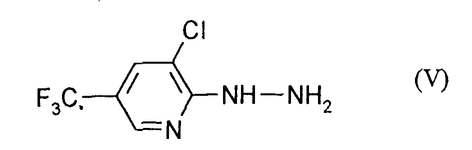 gegebenenfalls in Gegenwart eines Verdünnungsmittels, wie beispielsweise Ethanol oder Eisessig und gegebenenfalls in Gegenwart eines Reaktionshilfsmittels, wie beispielsweise Schwefelsäure, bei Temperaturen zwischen 20°C und 100°C erhitzt (vgl. EP-A 201 852 und Herstellungsbeispiel).

Die Verbindungen der Formeln (IV) und (V) sind allgemein bekannte Verbindungen der organischen Chemie.

Die zur Durchführung des erfindungsgemäßen Verfahrens (a) außerdem benötigten Trifluormethylsulfenylhalogenide der Formel (III) sind ebenfalls allgemein bekannte Verbindungen der organischen Chemie.

Das zur Durchführung des erfindungsgemäßen Verfahrens (b) als Ausgangsstoff benötigte 5-Amino-3-methyl-4-trifluormethylthio-[(3-chlor-5-trifluormethyl)-2-pyridyl]-pyrazol der Formel (Ia) ist neu und Gegenstand der Erfindung. Es ist erhältlich nach Verfahren (a).

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril; Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Säuren, wie beispielsweise Essigsäure.

Das erfindungsgemäße Verfahren (a) kann gegebenenfalls in Gegenwart eines Reaktionshilfsmittels durchgeführt werden. Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen -20°C und +120°C, vorzugsweise bei Temperaturen zwischen 0°C und +50°C.

Zur Durchführung des erfindungsgemäßen Verfahrens (b) setzt man pro Mol Pyridylpyrazol der Formel (II) im allgemeinen 1,0 bis 2,5 Mol, vorzugsweise 1,0 bis 1,5 Mol an Sulfenylhalogenid der Formel (III) und gegebenenfalls 1,0 bis 2,5 Mol, vorzugsweise 1,0 bis 1,5 Mol an Reaktionshilfsmittel ein. Die Reaktionsführung, Aufarbeitung und Isolierung der Reaktionsprodukte der Formel (Ia) erfolgt nach allgemein üblichen Verfahren.

Als Verdünnungsmittel zur Durchführung des erfindungsgemäßen Verfahrens (b) kommen ebenfalls inerte organische Lösungsmittel infrage. Vorzugsweise verwendet man Kohlenwasserstoffe, wie Benzin, Benzol, Toluol, Hexan oder Petrolether; chlorierte Kohlenwasserstoffe, wie Dichlormethan, 1,2-Dichlorethan, Chloroform, Tetrachlorkohlenstoff oder Chlorbenzol; Ether, wie Diethylether, Dioxan oder Tetrahydrofuran; Carbonsäuren, wie Essigsäure oder Propionsäure, oder dipolare aprotische Lösungsmittel, wie Acetonitril, Aceton, Essigsäureethylester oder Dimethylformamid.

Das erfindungsgemäße Verfahren (b) kann gegebenenfalls in Gegenwart eines Säurebindemittels durchgeführt werden. Als solche kommen alle üblicherweise verwendbaren organischen und anorganischen Säurebindemittel infrage. Vorzugsweise verwendet man Erdalkali- oder Alkalimetallhydroxide, -acetate oder - carbonate, wie beispielsweise Calciumhydroxid, Natriumhydroxid, Natriumacetat oder Natriumcarbonat.

Das erfindungsgemäße Verfahren (b) kann gegebenenfalls in Gegenwart eines geeigneten Katalysators durchgeführt werden. Als solche kommen alle üblicherweise für derartige Schwefeloxidationen gebräuchlichen Metallsalz-Katalysatoren infrage. Beispielhaft genannt sei in diesem Zusammenhang Ammoniummolybdat und Natriumwolframat.

Die Reaktionstemperaturen können bei der Durchführung des erfindungsgemäßen Verfahrens (b) in einem größeren Bereich variiert werden. Im allgemeinen arbeitet man bei Temperaturen zwischen -20°C und +70°C, vorzugsweise bei Temperaturen zwischen 0°C und +50°C.

Zur Durchführung des erfindungsgemäßen Verfahrens (b) setzt man pro Mol Pyridylpyrazol der Formel (Ia) im allgemeinen 0,8 bis 1,2 Mol, vorzugsweise äquimolare Mengen Oxidationsmittel ein, wenn man die Oxidation des Schwefels auf der Sulfoxidstufe unterbrechen will. Zur Oxidation zum Sulfon setzt man pro Mol substituiertem Pyridylpyrazol der Formel (Ia) im allgemeinen 1,8 bis 3,0 Mol, vorzugsweise doppelt molare Mengen an Oxidationsmittel ein. Die Reaktionsdurchführung, Aufarbeitung und Isolierung der Endprodukte der Formel (Ib) erfolgt nach üblichen Verfahren.

Als Oxidationsmittel zur Durchführung des erfindungsgemäßen Verfahrens (b) kommen alle üblichen zur Schwefeloxidation verwendbaren Oxidationsmittel infrage. Insbesondere geeignet sind Wasserstoffperoxid, organische Persäuren, wie beispielsweise Peressigsäure, m-Chlorperbenzoesäure, p-Nitroperbenzoesäure oder Luftsauerstoff.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec..

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pediculus humanus corporis, Haematopinus spp., Linognathus spp., Phthirus spp., Solenopotes spp..

Aus der Ordnung der Mallophaga z.B. Trichodectes spp., Damalinea spp., Baricola spp., Felicola spp., Columbicula spp..

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp..

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Macrosiphum avenae, Myzus spp., Pemphigus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp..

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp.. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Bruchidius obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Haematobia spp., Chrysops spp., Hydrotaca spp., Phlebotomus spp., Latromyia spp.

Aus der Ordnung der Siphonaptera z.B. Xenopsylla cheopis, Ceratophyllus spp., Polex spp., Ctenocephalides spp., Echichrophaga spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe wirken nicht nur gegen Pflanzen-, Hygiene - und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer.

Sie sind gegen normalsensible und resistente Arten und Stämme sowie gegen alle parasitierenden und nicht parasitierenden Entwicklungsstadien der Ekto- und Endoparasiten wirksam.

Die erfindungsgemäßen Wirkstoffe zeichnen sich durch eine hohe insektizide und akarizide Wirksamkeit aus.

Sie lassen sich mit besonders gutem Erfolg zur Bekämpfung von parasitisch lebenden Warmblüterschädlingen, wie beispielsweise gegen die Larven der Goldfliege (Lucilia cuprina), gegen Rinderzecken (Boophilus microplus), gegen Räudemilben (Psoroptes ovis), gegen Schaben (Blattella germanica u.a.), gegen Fliegen (Musca domestica) sowie gegen Flöhe (Ctenophalides felis) einsetzen.

Weiterhin zeigen die erfindungsgemäßen Verbindungen eine Wirksamkeit gegenüber parasitischen Protozoen und zwar insbesondere gegen Coccidien, Plasmodium sowie gegen pflanzenschädigende Insekten und Milben.

Die Wirkstoffe eignen sich bei günstiger Warmblütertoxizität zur Bekämpfung von tierischen Schädlingen wie Arthropoden, vorzugsweise Insekten und Spinnentieren, die in der Tierhaltung und Tierzucht bei Haus- und Nutztieren sowie Zoo-, Labor-, Versuchs- und Hobbytieren vorkommen. Sie sind dabei gegen alle oder einzelne Entwicklungsstadien der Schädlinge sowie gegen resistente und normal sensible Arten der Schädlinge wirksam.

Durch die Bekämpfung der tierischen Schädlinge sollen Krankheiten und deren Übertragung, Todesfälle und Leistungsminderungen (z.B. bei der Produktion von Fleisch, Milch, Wolle, Häuten, Eiern) verhindert werden, so daß durch den Einsatz der Wirkstoffe eine wirtschaftlichere und einfachere Tierhaltung möglich ist bzw. in bestimmten Gebieten erst möglich wird.

Zu den Schädlingen gehören:
Aus der Ordnung der Anoplura z.B. Haematopinus spp., Linognathus spp., Solenopotes spp., Pediculus spp., Pthirus spp.;
aus der Ordnung der Mallophaga z.B. Trimenopon spp., Menopon spp., Eomenacanthus spp., Menacanthus spp., Trichodectes spp., Felicola spp., Damalinea spp., Bovicola spp.;
aus der Ordnung der Diptera z.B. Chrysops spp., Tabanus spp., Musca spp., Hydrotaea spp., Muscina spp., Haematobasca spp., Haematobia spp., Stomoxys spp., Fannia spp., Glossina spp., Luculia spp., Calliphora spp., Auchmeromyia spp., Cordylobia spp., Cochliomyia spp., Chrysomyia spp., Sarcophaga spp., Wohlfartia spp., Gasterophilus spp., Oesteromyia spp., Oedemagena spp., Hypoderma spp., Oestrus spp., Rhinoestrus spp., Melophagus spp., Hippobosca spp.

Aus der Ordnung der Metastigmata z.B. Hyalomma spp., Rhipicephalus spp., Boophilus spp., Amblyomma spp., Haemophysalis spp., Dermacentor spp., Ixodes spp., Argas spp., Ornithodorus spp., Otobius spp.;
aus der Ordnung der Mesastigmata z.B. Dermanyssus spp., Ornithonyssus spp., Pneumonyssus spp..

Aus der Ordnung der Prostigmata z.B. Cheyletiella spp., Psorergates spp., Myobia spp., Demodex spp., Neotrombicula spp.;
aus der Ordnung der Astigmata z.B. Acarus spp., Myocoptes spp., Psoroptes spp., Chorioptes spp., Otadectes spp., Sarcoptes spp., Notoedres spp., Knemidocoptes spp., Necknemidocoptes spp., Lytodites spp., Laminosioptes spp..

Zu den Haus- und Nutztieren gehören Säugetiere wie z.B. Rinder, Schafe, Ziegen, Pferde, Schweine, Hunde, Katzen, Kamele, Wasserbüffel, Esel, Kaninchen, Damwild, Rentiere, Pelztiere wie z.B. Nerze, Chinchilla, Waschbär, Vögel wie z.B. Hühner, Puten, Fasane, Gänse, Enten.

Zu Labor- und Versuchstieren gehören z.B. Mäuse, Ratten, Meerschweinchen, Goldhamster, Hunde und Katzen.

Zu den Hobbytieren gehören z.B. Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Boli, Tränken, Granulaten, oral applizierbare Lösungen, Suspensionen oder Emulsionen, medikiertem Futter oder Trinkwasser. Die dermale Anwendung geschieht z.B. in Form des Tauchens (Dippen), Sprühens (Sprayen) oder Aufgießens (pour-on und spot-on) und des Einpuderns. Die parenterale Anwendung geschieht z.B. in Form der Injektion (z.B. intramusculär, subcutan, intravenös) oder durch Implantate.

Besonders hervorgehoben seien die Zubereitungen zur dermalen Anwendung. Dazu gehören Lösungen, Suspensions- und Emulsionskonzentrate sowie Mikroemulsionen, die vor Anwendung mit Wasser verdünnt werden, Aufgußformulierungen, Pulver und Stäube, Aerosole und wirkstoffhaltige Formkörper sowie dust-bags, back-rubber.

Zu den oberflächenaktiven Stoffen zählen:
Emulgatoren und Netzmittel wie anionaktive Tenside, z.B. Alkylsulfonate, Alkylsulfate, Arylsulfonate, Na-Laurylsulfate, Fettalkoholethersulfate, Mono/Dialkylpolyglykoletherorthophosphorsäureester-Monoethanolaminsalz, Calciumalkylarylsulfonat;
kationaktive Tenside, z.B. Cetyltrimethylammoniumchlorid;
ampholytische Tenside, z.B. Di-Na-N-lauryl-beta-iminodipropionat oder Lecithin;
nichtionogene Tenside, z.B. polyoxyethyliertes Ricinusöl, polyoxyethyliertes Sorbitan-Monooleat, polyoxyethyliertes Sorbitan-Monostearat, Glycerinmonostearat, Polyoxyethylenstearat, Alkylphenolpolyglykolether, polyoxyethyliertes Sorbitanmonopalmitat, Polyoxyethylenlaurylether, Polyoxyethylen-oleylether, Polyoxyethylenmannitanmonolaurat, Alkylpolyglykolether, Oleylpolyglykolether, Dodecylpolyglykolether, ethoxyliertes Nonylphenol, Isooctylphenolpolyethoxyethanol.

Die Zubereitungen können außerdem enthalten:
Haftvermittler, z.B. Carboxymethylcellulose, Methylcellulose und andere Cellulose- und Stärke-Derivate, Polyacrylate, Alginate, Gelatine, Gummi arabicum, Polyvinylpyrrolidon, Polyvinylalkohol, Copolymere aus Methylvinylether und Maleinsäureanhydrid, Polyethylenglykole, Paraffine, Öle, Wachse, hydriertes Rizinusöl, Lecithine und synthetische Phospholipide.

Die Zubereitungen können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe enthalten.

Die Zubereitungen können Spreitmittel enthalten, z.B. Silikonöle verschiedener Viskosität, Fettsäureester wie Ethylstearat, Di-n-butyl-adipat, Laurinsäurehexylester, Dipropylen-glykolpelargonat, Ester einer verzweigten Fettsäure mittlerer Kettenlänge mit gesättigten Fettalkoholen C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester, Dibutylphthalat, Adipinsäurediisopropylester, letzterem verwandte Estergemische u.a.;
Triglyceride wie Capryl/Caprinsäuretriglycerrid, Triglyceridgemische mit Pflanzenfettsäuren der Kettenlänge C₈-C₁₂ oder anderen speziell ausgewählten natürlichen Fettsäuren, Partialglyceridgemische gesättigter oder ungesättigter evtl. auch hydroxylgruppenhaltige Fettsäuren, Mono/Diglyceride der C₈/C₁₀-Fettsäuren und andere;
Fettalkohole wie Isotridecylalkohol, 2-Octyldodecanol, Cetylstearylalkohol, Oleylalkohol.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind gegebenenfalls gebrochene und fraktionierte, z.B. synthetische und natüliche Gesteinsmehle wie Kaoline, Talkum, Kreide, Quarz, Diatomeenerde, Kochsalz, Carbonate wie Calciumcarbonat, Hydrogencarbonate, Aluminiumoxide, Kieselsäuren, Tonerden, gefälltes oder kolloidales Siliciumdioxid, Phospate.

Organische Stoffe sind z.B. Zucker, Zellulose, Nahrungs- und Futtermittel wie Milchpulver, Tiermehle, Getreidemehle und -schrote, Stärken, Sägemehl.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in Form ihrer oben erwähnten festen oder flüssigen Formulierungen auch eingekapselt vorliegen.

Die Wirkstoffe können in Form ihrer oben erwähnten festen oder flüssigen Formulierungen auch eingekapselt vorliegen.

Die Wirkstoffe können auch in Form eines Aerosols angewendet werden. Dazu wird der Wirkstoff in geeigneter Formulierung unter Druck fein verteilt.

Es kann auch vorteilhaft sein, die Wirkstoffe in Formulierungen anzuwenden, die den Wirkstoff verzögert freigeben. Als solche seien wirkstoffhaltige Formkörper wie z.B. Platten, Bänder, Streifen, Halsbänder, Ohrmarken, Schwanzmarken, Gließmaßenbänder, Halfter, Markierungsvorrichtungen genannt. Als solche seien auch wirkstoffhaltige Implantate und Boli genannt.

Die Verabreichung der Wirkstoffe kann auch zusammen mit dem Futter und/oder dem Trinkwasser erfolgen.

Die Wirkstoffe können in den Formulierungen allein oder in Mischung mit anderen Wirkstoffen oder Synergisten vorliegen.

Direkt angewendete Formulierungen enthalten zwischen 10⁻⁷ und 5 Gew.-%, bevorzugt zwischen 10⁻⁴ und 1 Gew.-% Wirkstoff.

Formulierungen die erst nach weiterer Verdünnung angewendet werden enthalten 1 bis 95 Gew.-%, bevorzugt 5 bis 90 Gew.-% Wirkstoff.

Die Wirkstoffe können in die übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer,Kobalt, Molybdän und Zink verwendet werden.

Die erfindungsgemäßen Wirkstoffe können in ihren handelsüblichen Formulierungen sowie in den aus den Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Genannt seien die folgenden Verbindungen:
Acrinathrin, Alphamethrin, Betacyfluthrin, Bifenthrin, Brofenprox, Cis-Resmethrin, Clocythrin, Cycloprothrin, Cyfluthrin, Cyhalothrin, Cypermethrin, Deltamethrin, Esfenvalerate, Etofenprox, Fenpropathrin, Fenvalerate, Flucythrinate, Fluvalinate, Lambda-Cyhalothrin, Permethrin, Pyresmethrin, Pyrethrum, Silafluofen, Tralomethrin, Zetamethrin,
Alanycarb, Bendiocarb, Benfuracarb, Bufencarb, Butocarboxim, Carbaryl, Cartap, Ethiofencarb, Fenobucarb, Fenoxycarb, Isoprocarb, Methiocarb, Methomyl, Metolcarb, Oxamyl, Pirimicarb, Promecarb, Propoxur, Terbam, Thiodicarb, Thiofanox, Trimethacarb, XMC, Xylylcarb,
   Acephate, Azinphos A, Azinphos M, Bromophos A, Cadusafos, Carbophenothion, Chlorfenvinphos, Chlormephos, Chlorpyrifos, Chlorpyrifos M, Cyanophos. Demeton M, Demeton-S-methyl, Demeton S, Diazinon, Dichlorvos, Dicliphos, Dichlorfenthion, Dicrotophos, Dimethoate, Dimethylvinphos, Dioxathion, Disulfoton, Edifenphos, Ethion, Etrimphos, Fenitrothion, Fenthion, Fonophos, Formothion, Heptenophos, Iprobenfos, Isazophos, Isoxathion, Phorate, Malathion, Mecarbam, Mevinphos, Mesulfenphos, Methacrifos, Methamidophos, Naled, Omethoate, Oxydemeton M, Oxydeprofos, Parathion A, Parathion M, Phenthoate, Phorate, Phosalone, Phosmet, Phosphamidon, Phoxim, Pirimiphos A, Pirimiphos M, Propaphos, Prothiophos, Prothoate, Pyraclophos, Pyridaphenthion, Quinalphos, Salithion, Sebufos, Sulfotep, Sulprofos, Tetrachlorvinphos, Temephos, Thiomethon, Thionazin, Trichlorfon, Triazophos, Vamidothion,
Buprofezin, Chlorfluazuron, Diflubenzuron, Flucycloxuron, Flufenoxuron, Hexaflumuron, Pyriproxifen, Tebufenozide, Teflubenzuron, Triflumuron,
Imidacloprid, Nitenpyram, N-[(6-Chloro-3-pyridinyl)methyl]-N'-cyano-N-methylethanimidamid (NI-25),
Abamectin, Amitrazin, Avermectin, Azadirachtin, Bensultap, Bacillus thuringiensis, Cyromazine, Diafenthiuron, Emamectin, Ethofenprox, Fenpyrad, Fipronil, Flufenprox, Lufenuron, Metaldehyd, Milbemectin, Pymetrozine, Tebufenpyrad, Triazuron,
Aldicarb, Bendiocarb, Benfuracarb, Carbofuran, Carbosulfan, Chlorethoxyfos, Cloethocarb, Disulfoton, Ethophrophos, Etrimphos, Fenamiphos, Fipronil, Fonofos, Fosthiazate, Furathiocarb, HCH, Isazophos, Isofenphos, Methiocarb, Monocrotophos, Nitenpyram, Oxamyl, Phorate, Phoxim, Prothiofos, Pyrachlofos, Sebufos, Silafluofen, Tebupirimphos, Tefluthrin, Terbufos, Thiodicarb, Thiafenox,
Azocyclotin, Butylpyridaben, Clofentezine, Cyhexatin, Diafenthiuron, Diethion, Emamectin, Fenazaquin, Fenbutatin Oxide, Fenothiocarb, Fenpropathrin, Fenpyrad, Fenpyroximate, Fluazinam, Fluazuron, Flucycloxuron, Flufenoxuron, Fluvalinate, Fubfenprox, Hexythiazox, Ivemectin, Methidathion, Monocrotophos, Moxidectin, Naled, Phosalone, Profenofos, Pyraclofos, Pyridaben, Pyrimidifen, Tebufenpyrad, Thuringiensin, Triarathene sowie 4-Bromo-2-(4-chlorophenyl)-1-(ethoxymethyl)-5-(trifluoromethyl)-1H-pyrrole-3-carbonitril (AC 303630).

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Der erfindungsgemäße Wirkstoff eignet sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung des erfindungsgemäßen Wirkstoffs geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke), sowie eine Anwendung in Form der sogenannten Umgebungsbehandlung möglich.
Die Herstellung und biologische Wirksamkeit der erfindungsgemäßen Verbindung soll anhand der folgenden Beispiele erläutert werden.

### Herstellungsbeispiele:

### Beispiel 1

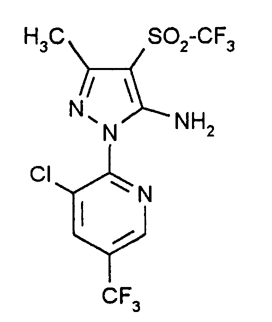

2,4 g (0,006 Mol) 5-Amino-3-methyl-4-trifluormethylthio-[(3-chlor-5-trifluormethyl)-2-pyridyl]-pyrazol (Bsp. 2) werden in 30 ml Essigsäure gelöst und mit einer Spatelspitze Natriumwolframat versetzt. Zu dieser Lösung werden bei Raumtemperatur tropfenweise 10 g (0,086 Mol) 30%-ige WasserstoffperoxidLösung zugesetzt. Es wird noch 18 Stunden nachgerührt. Danach wird das Reaktionsgemisch mit ca. 100 ml Wasser verdünnt. Die Ausfällung wird abfiltriert und getrocknet.

Man erhält 1,4 g (54 % der Theorie) 5-Amino-3-methyl-4-trifluormethylsulfonyl-[(3-chlor-5-trifluormethyl)-2-pyridyl]-pyrazol als hellgelben Feststoff vom Schmelzpunkt 93°C.

### Beispiel 2

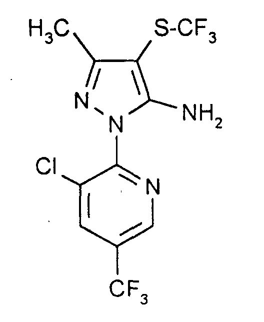

6,6 g (0,024 Mol) 5-Amino-3-methyl-[(3-chlor-5-trifluormethyl)-2-pyridyl]-pyrazol werden in 60 ml absolutem Dichlormethan gelöst und mit 2,1 g (0,026 Mol) absolutem Pyridin versetzt. Dann kühlt man auf 0-5°C ab und tropft 3,6 g (0,026 Mol) Trifluormethyl-sulfenylchlorid zu. Man rührt 3 Stunden bei 0°C und dann über Nacht bei Raumtemperatur. Anschließend wäscht man 2 mal mit Wasser, trocknet mit Magnesiumsulfat und zieht das Lösungsmittel im Vakuum ab.

Man erhält 6 g (67 % der Theorie) 5-Amino-3-methyl-4-trifluormethylthio-[(3-chlor-5-trifluormethyl)-2-pyridyl]-pyrazol als rotbraunes Wachs.

### Herstellung des Ausgangsproduktes

### Beispiel (II)

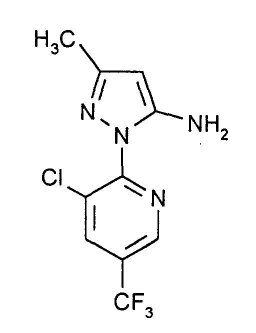

12 g (0,057 Mol) 3-Chlor-5-(Trifluormethyl)-pyrid-2-ylhydrazin und 4,7 g (0,057 Mol) 3-Aminocrotonitril werden in 100 ml Ethanol und 1 ml konzentrierter Schwefelsäure 24 Stunden unter Rückfluß erhitzt. Danach werden nochmals 4 ml konzentrierte Schwefelsäure zugegeben und für weitere 8 Stunden bei 60°C gerührt. Anschließend wird das Lösungsmittel im Vakuum abgesaugt und der orangefarbene Rückstand in Wasser und Dichlormethan aufgenommen. Die Dichlormethanphase wird abgetrennt, über Magnesiumsulfat getrocknet und das Lösungsmittel im Vakuum entfernt.

Man erhält 7,8 g (49 % der Theorie) 5-Amino-3-methyl-[(3-chlor-5-trifluormethyl)-2-pyridyl]-pyrazol als zähes rotbraunes Öl.

### Beispiel 3:

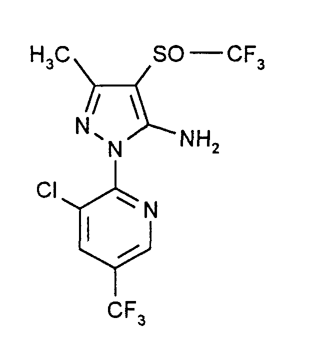

9 g (0,024 Mol) 5-Amino-3-methyl-4-trifluormethylthio-[(3-chlor-5-trifluormethyl)-2-pyridyl]-pyrazol (Bsp. 2) werden in 50 ml Dichlormethan gelöst und portionsweise mit 8,5 g (0,027 Mol) 55 %-iger m-Chlorperbenzoesäure versetzt. Es wird noch 48 Stunden bei Raumtemperatur gerührt. Danach wird der Niederschlag abfiltriert und verworfen. Das Filtrat wird mit Natriumcarbonat-Lösung und anschließend mit verdünnter Natronlauge gewaschen. Die organische Phase wird nach dem Trocknen über Magnesiumsulfat im Vakuum eingeengt. Es verbleiben 6,2 g rotes Harz als Rückstand, der über etwa 400 g Kieselgel 60 chromatographiert wird. Mit Cyclohexan/Essigsäureethylester (2:1) als Laufmittel erhält man 1,9 g (20 % der Theorie) an 5-Amino-3-methyl-4-trifluormethylsulfinyl-[(3-chlor-5-trifluormethyl)-2-pyridyl]-pyrazol als zähes oranges Öl.

¹H-NMR Daten^{*):}
9,0 ppm (d, 1H); 8,82 ppm (d, 1H); 6,9 ppm (bs, NH₂); 2,2 ppm (s, 3H).
* ⁾ Die ¹H-NMR-Spektren wurden in Deuterochloroform (CDCl₃) oder Hexadeuterodimethylsulfoxid (DMSO-d₆) mit Tetramethylsilan (TMS) als innerem Standard aufgenommen. Angegeben ist die chemische Verschiebung als δ-Wert in ppm.

### Anwendungsbeispiele

In den folgenden Anwendungsbeispielen wird die nachstehend aufgeführte Verbindung als Vergleichssubstanz eingesetzt:

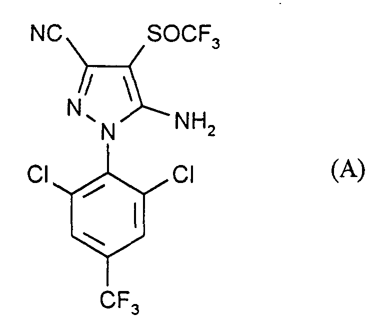

5-Amino-3-cyano-4-trifluormethyl-sulfinyl-1-[2,6-dichlor-4-(trifluormethyl)-phenyl]-pyrazol
(bekannt aus EP-A 295 117)

### Beispiel A

### Prüfung der Residualwirkung

Um die Wirkung von Wirkstoffen zu ermitteln, wurden verschiedene Unterlagen, wie z.B. PVC-Fußbodenbelag, unglasierte Kacheln, gebrannter Ton, Ton + Ca(OH)₂ und Sperrholz mit Präparaten, als wettable powder (WP) formuliert, in wässrigen Suspensionen in bestimmten Anwendungsmengen (mg a.i./m²) besprüht.

Eine Woche nach Behandlung bis zu 4 Wochen wöchentlich, wurden 10 Schaben der Art Blattella germanica im 5. Larvenstadium und 20 weibliche Stubenfliegen der Art Musca domestica auf die jeweilige Unterlage gesetzt. Die Schaben wurden innerhalb talkumierter Glasringe Fliegen mittels Käfigen aus Drahtgaze auf den behandelten Flächen gehalten und verblieben dort für 24 Stunden.

Die Auswertung auf prozentuale Abtötung erfolgte vom Zeitpunkt des Ansetzens aus gerechnet nach 15 und 30 Minuten, sowie nach einer bis zu 6 Stunden stündlich. Weitere Auswertungen erfolgten nach 8 und 24 Stunden.

In diesem Beispiel zeigt z.B. die erfindungsgemäße Verbindung (1) auf verschiedenen Oberflächen, wie beispielsweise PVC, Holz, unglasierte Kachel, Ton, Ton und Kalk als 10 %-iges WP mit 1000 mg a.i./m² eine erheblich bessere Residualwirkung als die aus dem Stand der Technik bekannte Verbindung (A).

### Beispiel B

### Blowfly-Larven Test

- Testobjekt:: Lucilia cuprina Larven
- Emulgator:: 35 Gewichtsteile Ethylenglykolmonomethylether 35 Gewichtsteile Nonylphenolpolyglykolether

Zur Herstellung einer zweckmäßigen Wirkstoffzubereitung vermischt man 3 Gew.-Teile Wirkstoff mit 7 Gew.-Teilen des oben angegebenen Gemisches und verdünnt das so erhaltene Emulsionskonzentrat mit Wasser auf die jeweils gewünschte Konzentration.

Etwa 20 Lucilia cuprina Larven (multiresistent) werden in ein Teströhrchen gebracht, welches ca. 1 cm³ Pferdefleisch und 0,5 ml der Wirkstoffzubereitung enthält. Nach 24 bis 48 h wird die Wirksamkeit der Wirkstoffzubereitung ermittelt. Dabei bedeutet 100 %, daß alle Blowfly-Larven abgetötet wurden; 0 % bedeutet, daß keine Blowfly-Larven abgetötet wurden.

Bei diesem Test zeigen die erfindungsgemäßen Verbindungen 1 und 2 bei einer Wirkstoffkonzentration von 100 ppm eine 100 %ige Wirkung gegen Lucilia cuprina, wohingegen der Stand der Technik keine Wirkung zeigt (0 %).

### Beispiel C

### In-vivo Test an Zecken/Spray am Rind

- Testobjekt:: Alle Stadien von Boophilus microplus (Larven, Metalarven, Nymphen, Metanymphen, Adulte), pyrethroid resistenter Stamm
- Emulgator:: 35 Gewichtsteile Ethylenglykolmonomethylether 35 Gewichsteile Nonylphenolpolyglykolether

Zur Herstellung einer zweckmäßigen Wirkstoffzubereitung vermischt man 3 Gew.-Teile Wirkstoff mit 7 Gew.-Teilen des oben angegebenen Gemisches und verdünnt das so erhaltene Emulsionskonzentrat mit Wasser auf die jeweils gewünschte Konzentration.

Rinder werden 14 x in 2-tägigem Abstand mit ca. 3000 nüchternen 14-28 Tage alten Larven von Boophilus microplus infiziert. Am 23. Tag p.i. wird das Rind mit 5 Litern der oben genannten Wirkstoffzubereitung gleichmäßig besprüht (Handspritze, 6 atü). Vom 24. bis zum 45. Tag p.i. werden die zur Entwicklung kommenden weiblichen Adulten gezählt, die Fertilität der Eigelege dieser Zecken kontrolliert und damit die Wirksamkeit der Wirkstoffzubereitung ermittelt. Dabei bedeutet 100 %, daß keine Zecken mit fertilen Eigelegen gefunden wurden; 0 % bedeutet, daß Anzahl der Zecken und Fertilität der Eigelege vergleichbar der Kontrolle waren.

In diesem Test zeigt die Verbindung 1 eine 100 %ige Wirkung gegen Boophilus microplus bei Wirkstoffkonzentrationen von 30 ppm und 100 ppm, wohingegen der Stand der Technik (A) bei 100 ppm lediglich 83 %ige Wirkung erreicht.

## Patentansprüche

1. Substituierte Pyridylpyrazole der allgemeinen Formel (I) 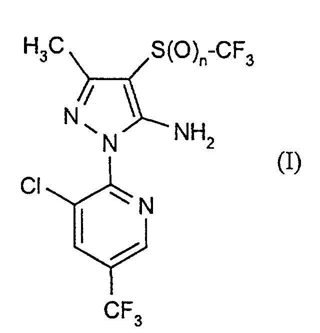 in welcher
n für die Zahl 0, 1 oder 2 steht.

2. Verfahren zur Herstellung substituierter Pyridylpyrazole gemäß Anspruch 1
**dadurch gekennzeichnet, daß** man
a) um 5-Amino-3-methyl-4-trifluormethylthio-[(3-chlor-5-trifluormethyl)-2-pyridyl]-pyrazol der Formel (Ia) 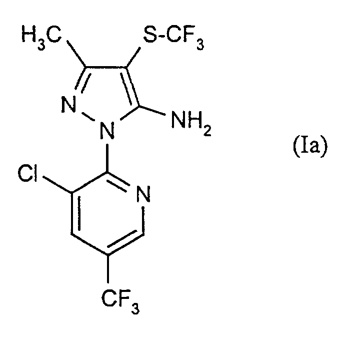 zu erhalten, 5-Amino-3-methyl-[(3-chlor-5-trifluormethyl)-2-pyridyl]-pyrazol der Formel (II) 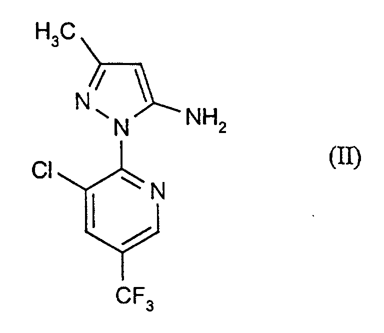 mit Trifluormethylsulfenylhalogeniden der Formel (III)
CF₃-S-Hal (III)
in welcher
Hal für Halogen, insbesondere Chlor oder Brom steht,
gegebenenfalls in Gegenwart eines Verdünnungsmittels und gegebenenfalls in Gegenwart eines Reaktionshilfsmittels umsetzt, oder daß man
b) um substituierte Pyridylpyrazole der Formel (Ib) 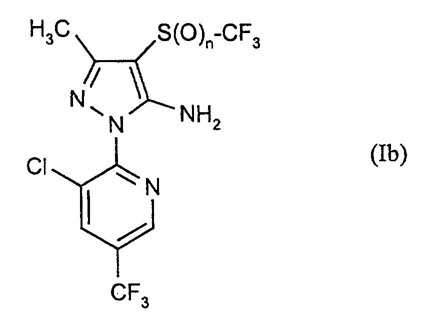 in welcher
n für die Zahl 1 oder 2 steht,
zu erhalten, 5-Amino-3-methyl-4-trifluormethylthio-[(3-chlor-5-trifluormethyl)-2-pyridyl]-pyrazol der Formel (Ia) 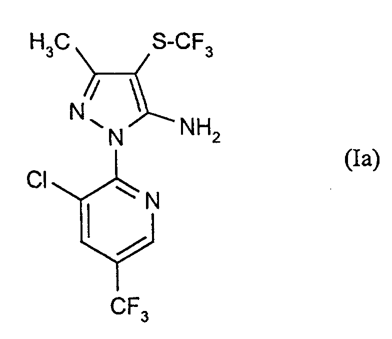 mit Oxidationsmitteln und in Gegenwart eines Verdünnungsmittels und gegebenenfalls in Gegenwart eines Katalysators oxidiert.

3. Schädlingsbekämpfungsmittel, **gekennzeichnet durch** einen Gehalt an mindestens einem substituierten Pyridylpyrazol gemäß Anspruch 1 und Streckmitteln und/oder oberflächenaktiven Mitteln.

4. Verfahren zur Bekämpfung von tierischen Schädlingen, insbesondere von Insekten, **dadurch gekennzeichnet, daß** man substituierte Pyridylpyrazole gemäß Anspruch 1 auf tierische Schädlinge und/oder ihren Lebensraum einwirken läßt.

5. Verwendung von substituierten Pyridylpyrazolen gemäß Anspruch 1 zur Bekämpfung von tierischen Schädlingen, insbesondere von Insekten.

6. Verfahren zur Herstellung von Mitteln gegen tierische Schädlinge, **dadurch gekennzeichnet, daß** man substituierte Pyridylpyrazole gemäß Anspruch 1 mit Streckmitteln und/oder oberflächenaktiven Mitteln vermischt.

## Claims

1. Substituted pyridylpyrazoles of the general formula (I) 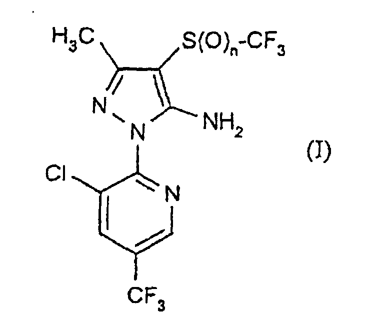 in which
n represents the number 0, 1 or 2.

2. Process for the preparation of substituted pyridylpyrazoles according to Claim 1,
**characterized in that**
a) to obtain 5-amino-3-methyl-4-trifluoromethylthio-[(3-chloro-5-trifluoromethyl)-2-pyridyl]-pyrazole of the formula (Ia) 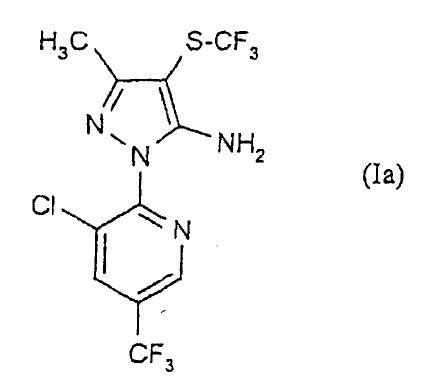 5-amino-3-methyl-[(3-chloro-5-trifluoromethyl)-2-pyridyl]-pyrazole of the formula (II) 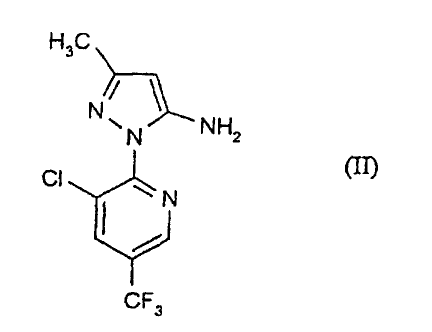 is reacted with trifluoromethylsulfenyl halides of the formula (III)
CF₃-S-Hal (III)
in which
Hal represents halogen, in particular chlorine or bromine,
if appropriate in the presence of a diluent and if appropriate in the presence of a reaction auxiliary, or **in that**
b) to obtain substituted pyridylpyrazoles of the formula (Ib) 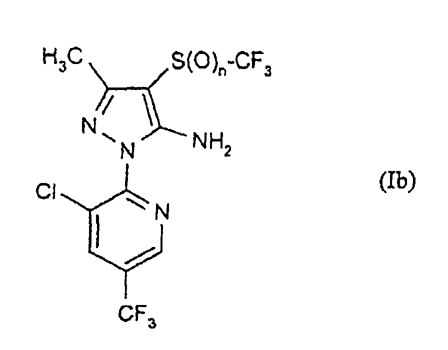 in which
n represents the number 1 or 2
5-amino-3-methyl-4-trifluoromethylthio-[(3-chloro-5-trifluoromethyl)-2-pyridyl]-pyrazole of the formula (Ia) 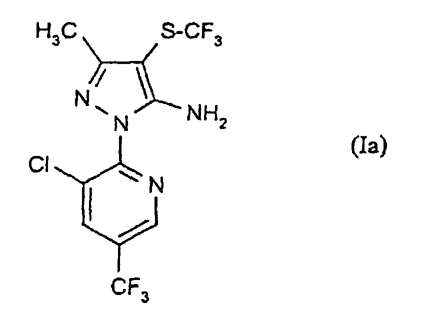 is oxidized with oxidants in the presence of a diluent and if appropriate in the presence of a catalyst.

3. Pesticide which comprises at least one substituted pyridylpyrazole according to Claim 1 and extenders and/or surfactants.

4. Method of controlling animal pests, in particular insects, **characterized in that** it comprises allowing substituted pyridylpyrazoles according to Claim 1 to act on animal pests and/or their environment.

5. Use of substituted pyridylpyrazoles according to Claim 1 for controlling animal pests, in particular insects.

6. Process for the preparation of compositions against animal pests, **characterized in that** it comprises mixing substituted pyridylpyrazoles according to Claim 1 with extenders and/or surfactants.

## Revendications

1. Pyridylpyrazoles substitués de formule générale (I) 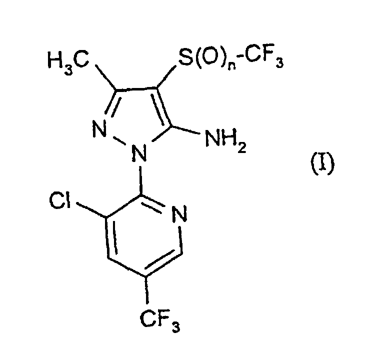 dans laquelle
n représente le nombre 0, 1 ou 2.

2. Procédé de production de pyridylpyrazoles substitués suivant la revendication 1, **caractérisé en ce que** :
a) pour obtenir le 5-amino-3-méthyl-4-trifluorométhylthio-[(3-chloro-5-trifluorométhyl)-2-pyridyl]-pyrazole de formule (Ia) 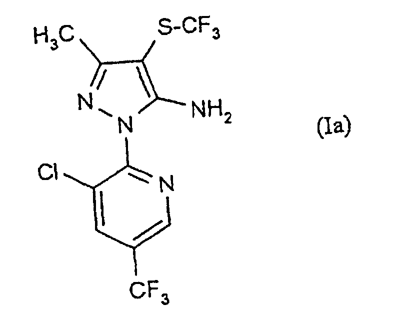 on fait réagir le 5-amino-3-méthyl-[(3-chloro-5-trifluorométhyl)-2-pyridyl]-pyrazole de formule (II) 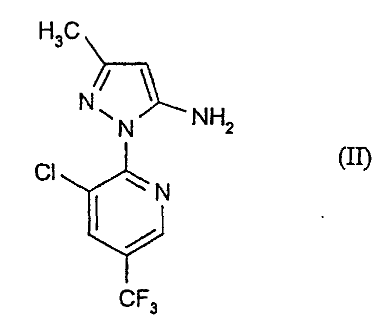 avec des halogénures de trifluorométhylsulfényle de formule (III)
CF₃-S-Hal (III)
dans laquelle
Hal représente un halogène, en particulier le chlore ou le brome,
le cas échéant en présence d'un diluant et en présence éventuelle d'un auxiliaire de réaction, ou bien
b) pour obtenir des pyridylpyrazoles substitués de formule (Ib) 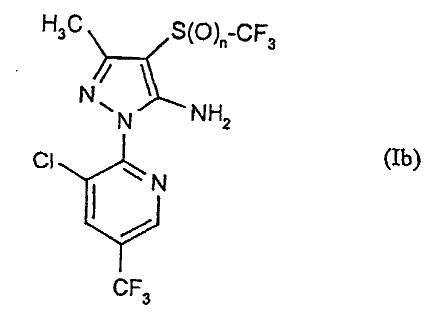 dans laquelle
n représente le nombre 0 ou 1,
on oxyde le 5-amino-3-méthyl-4-trifluorométhylthio-[(3-chloro-5-trifluorométhyl)-2-pyridyl]-pyrazole de formule (Ia) 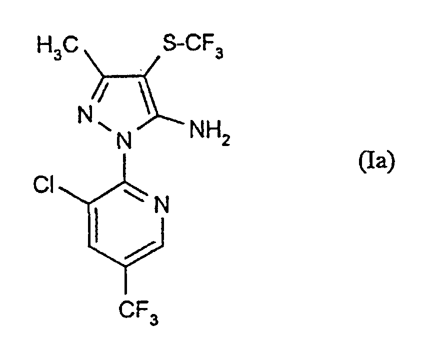 avec des agents oxydants et en présence d'un diluant et, le cas échéant, en présence d'un catalyseur.

3. Compositions pesticides, **caractérisées par** une teneur en au moins un pyridylpyrazole substitué suivant la revendication 1 avec des diluants et/ou des agents tensio-actifs.

4. Procédé pour combattre des parasites animaux, en particulier des insectes, **caractérisé en ce qu'**on fait agir des pyridylpyrazoles substitués suivant la revendication 1 sur les parasites animaux et/ou sur leur milieu.

5. Utilisation de pyridylpyrazoles substitués suivant la revendication 1 pour combattre des parasites animaux, en particulier des insectes.

6. Procédé de préparation de compositions contre des parasites animaux, **caractérisé en ce qu'**on mélange des pyridylpyrazoles substitués suivant la revendication 1 avec des diluants et/ou des agents tensio-actifs.
